(19) Europäisches Patentamt
European Patent Office
Office européen des brevets

(11) EP 2 997 979 A1

(12) **EUROPEAN PATENT APPLICATION**
published in accordance with Art. 153(4) EPC

(43) Date of publication:
**23.03.2016 Bulletin 2016/12**

(21) Application number: **14797011.5**

(22) Date of filing: **16.05.2014**

(51) Int Cl.:
***A61K 47/34*** *(2006.01)* ***A61K 9/107*** *(2006.01)*

(86) International application number:
**PCT/JP2014/063020**

(87) International publication number:
**WO 2014/185504 (20.11.2014 Gazette 2014/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.05.2013 JP 2013105465**

(71) Applicant: **NanoCarrier Co., Ltd.**
**226-39 Wakashiba**
**Kashiwa**
**Chiba 277-0871 (JP)**

(72) Inventors:
- **KATO, Yasuki**
  **Kashiwa-shi**
  **Chiba 277-0871 (JP)**
- **HARADA, Mitsunori**
  **Kashiwa-shi**
  **Chiba 277-0871 (JP)**
- **TANAKA, Ryosuke**
  **Kashiwa-shi**
  **Chiba 277-0871 (JP)**

(74) Representative: **Müller-Boré & Partner Patentanwälte PartG mbB**
**Friedenheimer Brücke 21**
**80639 München (DE)**

(54) **POLYMERIC MICELLE PHARMACEUTICAL COMPOSITION**

(57) A polymer micelle pharmaceutical composition is provided and includes: a block copolymer unit α having a hydrophilic polymer chain segment and a hydrophobic polymer chain segment; and a block copolymer unit β having a hydrophilic polymer chain segment and a hydrophobic polymer chain segment, wherein: the block copolymer unit α and the block copolymer unit β are radially arranged in the state in which the hydrophilic polymer chain segments are directed outward and the hydrophobic polymer chain segments are directed inward; and the hydrophobic polymer chain segment of the block copolymer unit α is constituted of repeating units having side chains , at least one of the side chains having a hydrophilic group.

FIG. 1

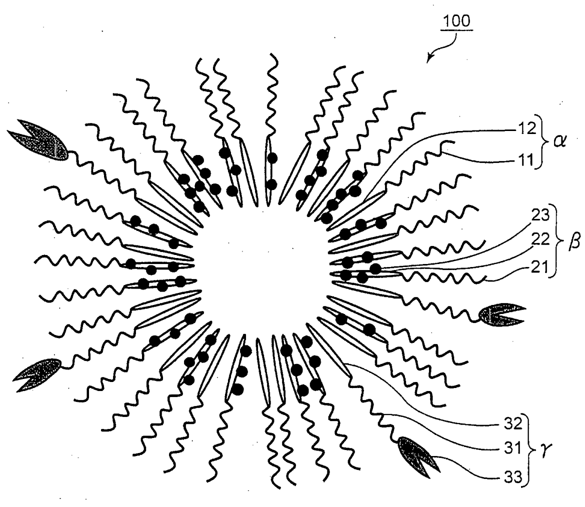


EP 2 997 979 A1

Printed by Jouve, 75001 PARIS (FR)

**Description**

TECHNICAL FIELD

The present invention relates to a polymer micelle pharmaceutical composition.

BACKGROUND ART

So far, various kinds of drug delivery systems (DDS) have been developed. For example, polymer micelle formulations utilizing a block copolymer having a hydrophilic segment and a hydrophobic segment have been developed (for example, Patent Literature 1 and Patent Literature 2). Such conventional polymer micelle formulation technology significantly contributes to enhancing drug efficacy while reducing side effects of drugs, but there is room for further improvement from the viewpoint of achieving higher levels of both.

Citation List

Patent Literature

Patent Literature 1: WO 2009/142326 A1
Patent Literature 1: WO 2010/013836 A1

SUMMARY OF THE INVENTION

Problem(s) to be Solved by the Invention

An object of the present invention is to provide a polymer micelle pharmaceutical composition having reduced side effects while exhibiting a high pharmacological effect.

Means for Solving the Problem(s)

In the field of the conventional polymer micelle technologies directed to the longer-term maintenance of the micelle particle structure in blood, from the viewpoint of strongly exhibiting hydrophobic interactionsbetween block copolymers, there is a material selection concept of setting as high as possible the degree of hydrophobicity of a hydrophobic segment moiety of the polymer. For example, there is a technological orientation for actively utilizing, as a micelle constituent material, a block copolymer having its polyamino acid segment side chains completely covered with hydrophobic structures such as benzyl groups (benzyl group introduction ratio: 100%). The orientation becomes particularly prominent when a non-drug-bound-type block copolymer is selected as a micelle constituent material.

The present inventors have found that, contrary to the above-described conventional material selection concept, when it is dared to apply a block copolymer (so-to-speak, a third-world block copolymer: third polymer), which weakens the contribution based on hydrophobic interactions to the strong maintenance of the micelle particle structure, as one component of the micelle constituent materials, the pharmacological effect of the drug to be delivered can be greatly enhanced while the drug side effect-suppressing effect of the polymer micelle technology is exhibited; thus, the inventors have completed the present invention.

That is, according to the present invention,

a polymer micelle pharmaceutical composition is provided and includes:
a block copolymer unit α having a hydrophilic polymer chain segment and a hydrophobic polymer chain segment; and
a block copolymer unit β having a hydrophilic polymer chain segment and a hydrophobic polymer chain segment, wherein:

the block copolymer unit α and the block copolymer unit β are radially arranged in the state in which the hydrophilic polymer chain segments are directed outward and the hydrophobic polymer chain segments are directed inward; and
the hydrophobic polymer chain segment of the block copolymer unit α is constituted of repeating units having side chains, at least one of the side chains having a hydrophilic group.

Effects of the Invention

According to the present invention, a polymer micelle pharmaceutical composition is provided, in which both of the enhancement of the pharmacological effect of the drug and the suppression of side effects become more remarkable.

BRIEF DESCRIPTION OF THE DRAWINGS

FIG. **1** is a schematic diagram of a polymer micelle pharmaceutical composition according to one embodiment of the present invention.

FIG. **2** is a graph that shows the results of a cellular uptake test for micelle formulations in Test Examples 1 to 6.

MODES FOR CARRYING OUT THE INVENTION

[1. Polymer Micelle Pharmaceutical Composition]

A polymer micelle pharmaceutical composition according to one embodiment of the present invention is described below. The polymer micelle pharmaceutical composition contains (a) block copolymer unit (s) $\alpha$ (polymer unit $\alpha$) and (a) block copolymer unit (s) $\beta$ (polymer unit $\beta$). The polymer unit (s) $\alpha$ has (have) a hydrophilic polymer chain segment **11** and a hydrophobic polymer chain segment **12.** The polymer unit(s) $\beta$ has (have) a hydrophilic polymer chain segment **21** and a hydrophobic polymer chain segment **22.** The polymer unit(s) $\alpha$ and the polymer unit(s) $\beta$ are radially arranged in the state in which the hydrophobic polymer chain segments **12** and **22** are each directed inward, and the hydrophilic polymer chain segments **11** and **21** are each directed outward. The hydrophobic polymer chain segment **12** of the polymer unit $\alpha$ is constituted of repeating units having side chains. At least one of the side chains has a hydrophilic group. The hydrophilic polymer chain segment of each of the polymer unit $\alpha$ and the polymer unit $\beta$ is typically a polyethylene glycol chain. The hydrophobic polymer chain segment of each of the polymer unit $\alpha$ and the polymer unit $\beta$ is typically a polyamino acid chain. It should be noted that the block copolymer unit $\alpha$ is hereinafter sometimes referred to as "backbone polymer unit $\alpha$" and the block copolymer unit $\beta$ is hereinafter sometimes referred to as "backbone polymer unit $\beta$." Further, any appropriate hydrophilic group may be utilized as the hydrophilic group herein. Examples of the hydrophilic group include a hydroxyl group, a carboxyl group, and an amino group.

In the present specification, to satisfy the condition that the block copolymer units are radially arranged, it is only required that the block copolymer units are in an aggregated state in which the hydrophobic polymer chain segments are directed inward and the hydrophilic polymer chain segments are directed outward; the micelle may have a slightly collapsed radially arranged structure, in which points of origin for the arrangement of the respective block copolymer units are not focused on one point. The polymer micelle pharmaceutical composition may be such that the polymer aggregate of the block copolymer units is in a dry state.

According to the above polymer micelle pharmaceutical composition, the therapeutic index can be greatly improved as compared to conventional polymer micelle formulations. Although the mechanism by which such effect is obtained is still unclear, it is presumed to be as follows. First, the polymer micelle has the property of being easily retained in the surroundings of a target cell by virtue of the EPR effect due to its particle size. Inaddi tion, it is considered that, in the polymer micelle pharmaceutical composition according to the present invention, the backbone polymer unit $\alpha$ is likely to be detached early from the micelle by virtue of the degree of hydrophilicity of its hydrophobic segment moiety. When the unit $\alpha$ is released from the micelle, which is retained in proximity of the target cell, the cell membrane of the target cell present in the vicinity of the retained micelle is easily stimulated by the hydrophobic groups of the hydrophobic segment moiety of the unit $\alpha$, resulting in activation of the endocytosis of the cell. As a result, it is considered that the delivery properties of the polymer micelle pharmaceutical composition including the drug to be delivered to the target cell are enhanced, and the pharmacological effect of the drug is more efficiently exhibited.

The polymer micelle pharmaceutical composition **100** may further include (a) block copolymer unit (s) $\gamma$ (hereinafter sometimes referredtoas "backbonepolymerunity") havingahydrophilicpolymer chain segment **31** having a target binding site **33** bound thereto, and a hydrophobic polymer chain segment **32.** The backbone polymer unit(s) $\gamma$ may be radially arranged together with the backbone polymer units $\alpha$ and $\beta$ in the state in which the hydrophilic polymer chain segment **31** is directed outward and the hydrophobic polymer chain segment **32** is directed inward.

In the present specification, the target binding site means a site having a biological recognition function, which can specifically bind to a substance originating from organisms and viruses and can form a biological binding pair with the substance. Examples of substances originating from organisms and viruses may include molecules present in

biological cells, bacteria, fungi, and viruses. Examples of the biological cells may include: tumor cells, neovascular cells, and surrounding cells thereof; immunocompetent cells (such as B cells); inflammatory cells (such as leukocytes) ; vascular endothelial cells; and cells constituting various organs. The target binding site may be formed in the state of containing, as at least part of its structure, a compound, such as a protein, a peptide, or a sugar chain, which forms a binding pair with such substance.

As aspects of the incorporation of the drug into the polymer micelle pharmaceutical composition, for example, an aspect in which the drug is encapsulated within themicelle, and for example, an aspect in which the drug is carried on the surface of the micelle, are given. Although the drug is typically bound to a block copolymer unit, it need not be bound to a block copolymer unit as long as the object of the present invention is not adversely affected. In case the drug is encapsulated in the micelle, it is appropriate that the site(s) at which the drug(s) is (are) bound to the block copolymer unit be a side chain and/or an inwardly projecting end of the hydrophobic polymer chain segment; in case the drug is carried on the surface of the micelle, it may be bound to an outwardly projecting end of the hydrophilic polymer chain segment. In case the drug is encapsulated in the micelle, the drug(s) is (are) typically bound to (a) side chain(s) of the repeating unit of the hydrophobic polymer chain segment. In the example illustrated in FIG. **1,** the drugs **23** are bound to the hydrophobic polymer chain segments **22** of the backbone polymer units β. Specific examples of the drug and the like are described below. It should be noted that the polymer micelle pharmaceutical composition according to the present invention typically contains the drug, but does not exclude the state of containing no drug. For example, the polymer micelle pharmaceutical composition according to the present invention may be in the state of containing the target binding site while containing no drug.

The polymer micelle pharmaceutical composition may be in the state of containing no target binding site. However, from the viewpoint of exhibiting the effect of the present invention at a higher level, the polymer micelle pharmaceutical composition is desirably in the state of containing the target binding site. This is because a polymer micelle retained in proximity of the target cell easily accumulates more locally toward the target cell by virtue of the EPR effect. As demonstrated in Test Examples to be described below as well, the polymer micelle pharmaceutical composition according to the present invention enables the delivery of the drug to be delivered to the target cell to an extent beyond the original ability of a compound serving as the target binding site to deliver the drug to the target cell (such as the ability of an antibody to be internalized in the cell membrane). FIG. **1** is an illustration of an aspect in which target binding sites **33** are bound to the surface of the polymer micelle pharmaceutical composition **100.** The target binding sites **33** are bound to the hydrophilic polymer chain segments **31** of the backbone polymer units y. It should be noted that the polymer micelle pharmaceutical composition according to the present invention does not exclude the state in which a target binding site is also bound to a block copolymer unit other than the backbone polymer unit γ.

The content of the backbone polymer unit α in the polymer micelle pharmaceutical composition is, for example, 15 wt% or more, for example, 20 wt% or more, and for example, 35 wt% or more. As demonstrated in Example and Test Examples to be described below, when the polymer micelle pharmaceutical composition contains a predetermined amount or more of the unit α, the delivery properties of the polymer micelle pharmaceutical composition to the target cell can be more certainly enhanced, and the pharmacological effect of the drug to be delivered can be greatly augmented. The upper limit of the content of the unit α is not particularly limited, but from the viewpoint of preventing excessive collapsing of the polymer micelle pharmaceutical composition, more specifically, from the viewpoint of securing the sustainability of the micelle structure until delivery to the vicinity of the target cell in blood while exhibiting the side effect-preventing action of the polymer micelle pharmaceutical composition, the upper limit maybe set to, for example, 80 wt%, for example, 60 wt%, for example, 50 wt%, and for example, 45 wt%.

The content of the backbone polymer unit β in the polymer micelle pharmaceutical composition is, for example, 80 wt% or less, for example, 70 wt% or less, and for example, 60 wt% or less. The lower limit of the content of the unit β is not particularly limited, but from the viewpoint of preventing an excessive decrease in the loading ratio of the drug to be delivered in the polymer micelle pharmaceutical composition, the lower limit maybe set to, for example, 15 wt%, for example, 25 wt%, and for example, 40 mass%. When the backbone polymer unit γ is contained in the polymer micelle pharmaceutical composition, its content maybe set as follows: (1) the lower limit is, for example, 1 wt%, for example, 3 wt%, for example, 5 wt%, and for example, 10 wt%; and (2) the upper limit is, for example, 20 wt%, and for example, 15 wt%.

In the polymer micelle pharmaceutical composition, the backbone polymer unit α and the backbone polymer unit β may be present at any appropriate ratio, and may be present at a molar ratio (former: latter) in, for example, the range of from 1:20 to 20:1, preferably the range of from 1: 10 to 10:1, more preferably the range of from 1:5 to 5:1, still more preferably the range of from 1:2 to 2:1.

The backbone polymer unit α and the backbone polymer unit β, and other backbone polymer units (such as the backbone polymer unit γ, and (a) backbone polymer unit (s) different from any of the backbone polymer units α, β, and γ) may be present at any appropriate ratio, and may be present at a molar ratio (total molar amount of backbone polymer units α and β : total molar amount of other backbone polymer units) in, for example, the range of from 100:0 to 100:300, preferably the range of from 100:1 to 100:100, more preferably the range of from 100:2 to 100:50.

The polymer micelle pharmaceutical composition 100 may include two or more kinds of block copolymer units

as each of the above-mentioned block copolymer units.

[2. Backbone Polymer Unit α]

The backbone polymer unit α has the hydrophilic polymer chain segment and the hydrophobic polymer chain segment.

The hydrophobic polymer chain segment of the backbone polymer unit α is constituted of repeating units having side chains. At least one of the side chains has a hydrophilic group. In the side chains of the hydrophobic polymer chain segment of the backbone polymer unit α, the percentage of side chains having a hydrophilic group is, for example, 20% or more, and for example, 35% or more. As demonstrated in Example and Test Examples to be described below, when the percentage of the side chains having a hydrophilic group is controlled to a predetermined amount or more, the delivery properties of the polymer micelle pharmaceutical composition to the target cell can be more certainly enhanced, and the pharmacological effect of the drug to be delivered can be greatly augmented. The upper limit of the percentage of the side chains having a hydrophilic group is not particularly limited, but from the viewpoint of preventing excessive collapsing of the polymer micelle pharmaceutical composition, the upper limit may be set to, for example, 80%, for example, 60%, and for example, 50%.

Although the mechanism by which the delivery properties of the polymer micelle pharmaceutical composition to the target cell can be more certainly enhanced through the control of the content of the backbone polymer unit α in the polymer micelle pharmaceutical composition and the ratio of the side chains each having a hydrophilic group in the side chains of the hydrophobic polymer chain segment to the above-mentioned ranges is still unclear, it is presumed, for example, to be as follows. As described above, it is considered that the backbone polymer unit α is likely to be detached early from the micelle by virtue of the degree of hydrophilicity of its hydrophobic segment moiety. It is considered that when the percentage of the side chains having a hydrophilic group in the side chains of the hydrophobic polymer chain segment of the unit α is controlled to the above-mentioned range, the early detachment of individual units α from the micelle can be more certainly induced. In addition, it is considered that when the content of the unit α in the micelle is controlled to the above-mentioned range, such an amount of the unit α as to be able to activate the endocytosis of the target cell at a high level can be more certainly detached early from the micelle. As a result, the delivery properties of the polymer micelle pharmaceutical composition, which includes the drug to be delivered to the target cell, are enhanced. It should be noted that even if a person skilled in the art of polymer micelles were to conceive of adding a non-drug-bound-type block copolymer as a micelle constituent material in order to improve the function of the micelle, he or she would generally have been inclined to keep its addition ratio as low as 10 mass% or less from the viewpoint of maintaining the drug loading ratio per micelle high. In this connection, as demonstrated in Test Examples to be described below, when the backbone polymer unit α is added at 10 mass%, the delivery properties to the target cell may be decreased contrarily as compared to the case of not adding the unit α. Accordingly, the addition ratio of the unit α is not to be further increased in the general technological orientation, whereas the inventors of the present invention have found the following hard-to-predict function-improving means: when the addition ratio of the unit α is further increased to the contrary, the delivery property of the polymer micelle pharmaceutical composition to the target cell is more certainly enhanced.

In the polymer micelle pharmaceutical composition 100, the backbone polymer unit α may be radially arranged in any appropriate state. For example, the backbone polymer unit α may be radially arranged in the state in which the hydrophilic polymer chain segment 11 is directed outward and the hydrophobic polymer chain segment 12 is directed inward.

The backbone polymer unit α desirably has a lower hydrophobicity than that of the backbone polymer unit β. The lower hydrophobicity may result from the hydrophilic group (s) of the side chains of the hydrophobic polymer chain segment.

When the hydrophobic polymer chain segment of the backbone polymer unit β is constituted of repeating units having side chains, the side chains of the hydrophobic polymer chain segment in the backbone polymer unit α may have a larger number of hydrophilic groups than the side chains of the hydrophobic polymer chain segment in the backbone polymer unit β.

Although the backbone polymer unit α is typically free of a target binding site and a drug, the state of containing a target binding site and/or a drug is not excluded.

The backbone polymer unit α may be a block copolymer represented by the general formula: $A_1$-$B_1$. $A_1$ represents a polyethylene glycol chain segment, and $B_1$ represents a polyamino acid chain segment having at least one side chain which has a hydrophilic group; typically it contains (an) amino acid residue (s) having a hydrophobic group in a side chain, and (an) amino acid residue(s) having a hydrophilic group in a side chain. It should be noted that polyethylene glycol is hereinafter sometimes denoted as "PEG".

Examples of the polyamino acid chain segment of the backbone polymer unit α having a hydrophobic group and a hydrophilic group may include: polyglutamic acid, or an ester or an amide derivative thereof; and polyaspartic

acid, or an ester or an amide derivative thereof. Such ester or amide derivative may be formed through a reaction between a corresponding hydroxy compound or amino compound having the hydrophobic group and the hydrophilic group, and a reactive derivative (such as an ester) of polyglutamic acid or polyaspartic acid.

Examples of the hydrophobic group include hydrophobic organic groups. Examples of the hydrophobic organic groups include a $C_4$ to $C_{16}$ alkyl group having a linear, branched, or cyclic structure, a $C_6$ to $C_{20}$ aryl group, and a $C_7$ to $C_{20}$ aralkyl group or sterol residue. Preferred examples of the $C_6$ to $C_{20}$ aryl group and the $C_7$ to $C_{20}$ aralkyl group include a phenyl group, a naphthyl group, a tolyl group, a xylyl group, a benzyl group, and a phenethyl group, and of those, a more preferred example thereof is a benzyl group. In addition, as a sterol from which the sterol residue results, cholesterol, cholestanol, and dihydroxycholesterol are preferred, and cholesterol is more preferred.

Preferred specific examples of the backbone polymer unit $\alpha$ may be represented by the following general formulae (I) and (II). The polymer micelle pharmaceutical composition of the present invention may include two or more kinds of the backbone polymer unit $\alpha$.

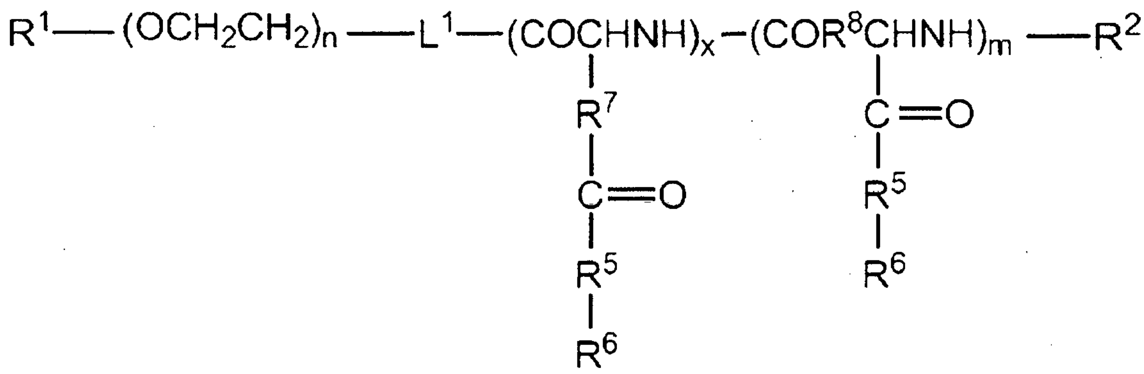

(I)

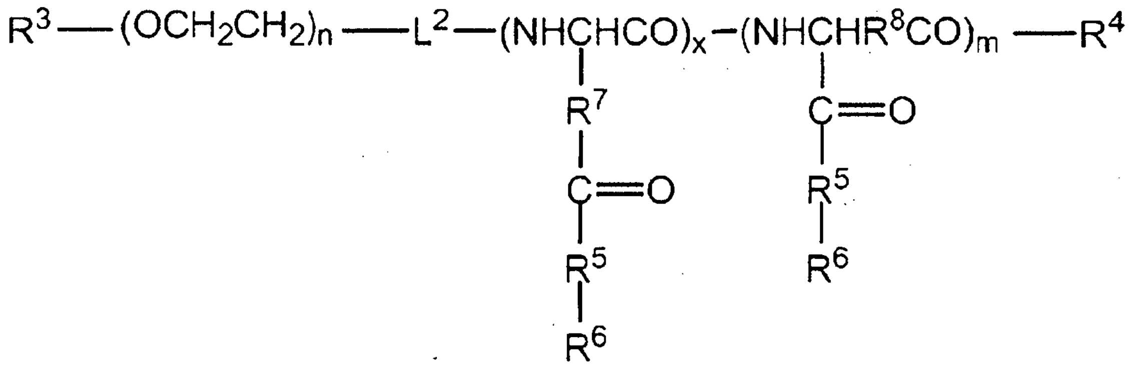

(II)

In each of the above-mentioned formulae:

$R^1$ and $R^3$ each independently represent a hydrogen atom or a lower alkyl group, which is unsubstituted or is substituted with an optionally protected functional group;
$R^2$ represents a hydrogen atom, a saturated or unsaturated $C_1$ to $C_{29}$ aliphatic carbonyl group, or an arylcarbonyl group;
$R^4$ represents a hydroxy group, a saturated or unsaturated $C_1$ to $C_{30}$ aliphatic oxy group, or an aryl-lower alkyloxy group;
$R^5$s each represent -O- or -NH-;
10% to 90% of the total number (m+x) of $R^6$s is/are (a) hydrogen atom(s), and the remaining $R^6$(s) is/are (a) hydrophobic organic group(s);
$R^7$ and $R^8$ each independently represent a methylene group or an ethylene group;
n is an integer of from 10 to 2,500;
x is an integer of from 10 to 300;
m is an integer of from 0 to 300 (provided that, when m is 1 or more, the binding order of the repeating units in which the number of repetitions is x and the repeating units in which the number of repetitions is m is arbitrary, and $R^6$s

are each independently selected in each repeating unit in one block copolymer);
$L^1$ represents a linking group selected from the group consisting of -NH-, -O-, -O-Z-NH-, -CO-, $-CH_2-$, -O-Z-S-Z-, and -OCO-Z-NH- (where Z's each independently represent a $C_1$ to $C_6$ alkylene group); and
$L^2$ represents a linking group selected from -OCO-Z-CO- and -NHCO-Z-CO- (where Z's each independently represent a $C_1$ to $C_6$ alkylene group).

1 The percentage of hydrogen atoms in the total number (m+x) of $R^6$s is, for example, 20% or more, and for example, 35% or more. The percentage is, for example, 80% or less, for example, 60% or less, and for example, 50% or less.
The above-mentioned n is preferably from 10 to 1,000, more preferably from 20 to 600, particularly preferably from 50 to 500. The above-mentioned x and m are each preferably from 20 to 200, more preferably from 30 to 100.
The binding order of the respective repeating units within the polyamino acid segment of the backbone polymer unit $\alpha$ represented by formulae (I) and (II) is arbitrary. The polyamino acid segment may have any of: a structure in which the binding order of the respective repeating units is random (random structure); a structure including a segment formed of repeating units with a number of repetitions of x and a segment formed of repeating units with a number of repetitions of m (block structure) ; and a structure in which m=0 (homopolymer structure).
Examples of the optionally protected functional group include a hydroxyl group, an acetal, a ketal, an aldehyde, a sugar residue, a maleimide group, a carboxyl group, an amino group, a thiol group, and an active ester. The hydrophilic polymer chain segment, in case $R^1$ and $R^3$ each represent a lower alkyl group substituted with an optionally protected functional group, may be determined, for example, in accordance with the methods described in WO 96/33233 A1, WO 96/32434 A1, and WO 97/06202 A1. The lower alkyl group means a linear or branched alkyl group having, for example, 7 or less, preferably 4 or less carbon atoms, such as a methyl group, an ethyl group, a propyl group, an isopropyl group, a butyl group, or an isobutyl group.
The backbone polymer unit $\alpha$ may be obtained, for example, by coupling a polymer having a hydrophilic polymer chain and a polymer having a polyamino acid chain by a known method, each of which has not been subjected to any treatment or has been purified so as to achieve a narrow molecular weight distribution as necessary. The block copolymer of the general formula (I) may also be formed, for example, by carrying out anionic living polymerization using an initiator capable of providing $R^1$ to form a polyethylene glycol chain, then introducing an amino group at the side of the growing end, and polymerizing an N-carboxylic anhydride (NCA) of a protected amino acid such as $\beta$-benzyl-L-aspartate or $\gamma$-benzyl-L-glutamate from the amino end.
A specific example of a method of manufacturing the backbone polymer unit $\alpha$ is described below. N-carboxy-$\beta$-benzyl-L-aspartate anhydride (BLA-NCA) or N-carboxy-$\gamma$-benzyl-L-glutamate anhydride (BLG-NCA) is added and subjected to reaction using, as an initiator, polyethylene glycol, which is protected at one end and has an amino group at the other end, such as $MeO-PEG-CH_2CH_2CH_2-NH_2$, in a dry organic solvent so as to achieve a desired degree of polymerization (number of amino acid units), whereby polyethylene glycol-co-polyaspartic acid benzyl ester or polyethylene glycol-co-polyglutamic acid benzyl ester may be prepared. In addition, the resultant block copolymer is acetylated at the end with acetyl chloride or acetic anhydride, then subjected to alkali hydrolysis to remove a benzyl group, and converted into polyethylene glycol-co-polyaspartic acid or polyethylene glycol-co-polyglutamic acid. After that, benzyl alcohol is added in an organic solvent so as to achieve a desired esterification ratio, and the reaction is carried out in the presence of a condensation agent such asN-N'-dicyclohexylcarbodiimide (DCC) or N-N'-diisopropyl carbodiimide (DIPCI), whereby a block copolymer partially having a benzyl ester may be prepared.
Further, when the reaction is performed using, for example, cholesterol in place of benzyl alcohol, polyethylene glycol-co-polyaspartic acid cholesterol ester and polyethylene glycol-co-polyglutamic acid cholesterol ester may be prepared.
Another specific example of a method of manufacturing the backbone polymer unit $\alpha$ is a method involving introducing a hydrophobic side chain through an amide bond. In the manufacturing method, polyethylene glycol-co-polyaspartic acid benzyl ester or polyethylene glycol-co-polyglutamic acidbenzyl ester is acetylated at the end in the same manner as described above. Then, a benzyl group is removed by alkali hydrolysis and the generated carboxyl group is subjected to reaction with a hydrophobic side chain having an amino group. Alternatively, polyethylene glycol-co-polyaspartic acid benzyl ester or polyethylene glycol-co-polyglutamic acid benzyl ester and a compound having a primary amine are subjected to reaction and then subjected to aminolysis to convert an ester bond to an amide bond. This allows the introduction of a hydrophobic side chain through an amide bond. In addition, a poly(amino acid derivative) segment including a mixture of a hydrophobic side chain having a hydrophobic group whose end has been substituted by an amino group and a hydrophobic side chain without amino group substitution may also be obtained by adding a primary amine such as 1-octylamine to polyethylene glycol-co-polyaspartic acid benzyl ester in an organic solvent so as to achieve a desired amidation ratio, subjecting the mixture to reaction for a predetermined period of time, and then adding a large excess amount of 1,8-diaminooctane or the like to an unconverted benzyl ester.
Other preferred specific examples of the backbone polymer unit $\alpha$ may be represented by the following general

formulae (III) and (IV).

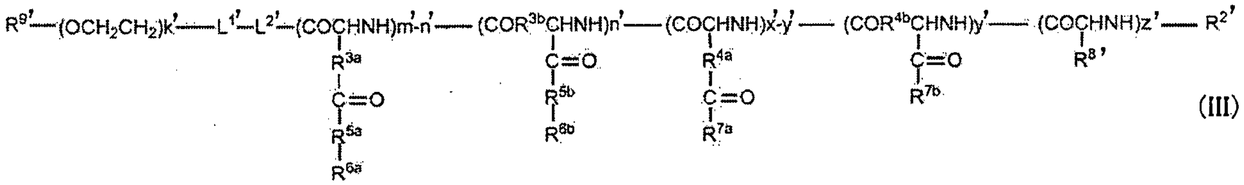

(III)

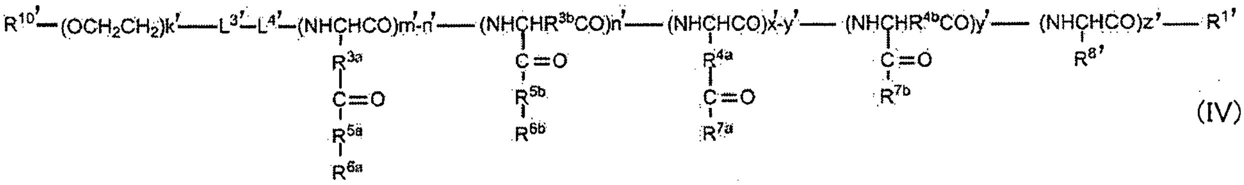

(IV)

In the formulae:

$R^{1'}$ represents a hydroxyl group, an unsubstituted or substituted linear or branched alkyloxy group having 1 to 12 carbon atoms, an unsubstituted or substituted linear or branched alkenyloxy group having 2 to 12 carbon atoms, an unsubstituted or substituted linear or branched alkynyloxy group having 2 to 12 carbon atoms, or an unsubstituted or substituted linear or branched alkyl-substituted imino group having 1 to 12 carbon atoms;
$R^{2'}$representsahydrogenatom, anunsubstitutedorsubstituted linear or branched alkyl group having 1 to 12 carbon atoms, or an unsubstituted or substituted linear or branched alkylcarbonyl group having 1 to 24 carbon atoms;
$R^{3a}$s, $R^{3b}$s, $R^4$as, and $R^{4b}$s each independently represent a methylene group or an ethylene group;
$R^{5a}$as and $R^{5b}$s each independently represent -O- or -NH-;
$R^{6a}$s and $R^{6b}$s each independently represent a saturated or unsaturated linear or branched aliphatic hydrocarbon group having 6 to 27 carbon atoms, an aromatic hydrocarbon group having 6 to 27 carbon atoms, or a steryl group;
$R^{7a}$s and $R^{7b}$s are each independently selected from groups identical to or different from each other in the group consisting of the following groups:

$-NH-(CH_2)_{p1}-[NH-(CH_2)q_1-]r_1NH_2$ (i) ;

$-NH-(CH_2)_{p2}-N[-(CH_2)_{q2}-NH_2]_2$ (ii) ;

$-NH-(CH_2)_{p3}-N\{[-(CH_2)_{q3}-NH_2][-(CH_2)_{q4}-NH-]_{r2}H\}$ (iii);

and

$-NH-(CH_2)_{p4}-N\{-(CH_2)_{q5}-N[-(CH_2)_{q6}-NH_2]_2\}_2$ (iv)

where p1 to p4, q1 to q6, and r1 to r2 are each independently an integer of from 1 to 5;
$R^{8'}$ represents a side chain of an amino acid selected from the group consisting of lysine, ornithine, arginine, homoarginine, and histidine;
m' is an integer of from 5 to 80;
n' is an integer of from 0 to m';
x' is an integer of from 0 to 20;
y' is an integer of from 0 to x';
z' is an integer of from 0 to 20,
provided that the total of x' and z' is 1 or more and 20 or less, the binding order of the respective repeating units is arbitrary, and $R^{6a}$s, $R^{6b}$s, $R^{7a}$s, $R^{7b}$s, and $R^8$'s each may be arbitrarily selected in each amino acid residue in one polyamino acid;
$L^{1'}$ and $L^{3'}$ are each independently -S-S- or a valence bond;
$L^{2'}$ is -NH-, -O-, $-O(CH_2)_{p5}-NH-$, or $-L^{2a}-(CH_2)_q7-L^{2b}-$ where p5 and q7 are each independently an integer of from 1 to 5, $L^{2a}$ is OCO, OCONH, NHCO, NHCOO, NHCONH, CONH, or COO, and $L^{2b}$ is NH or O;
$L^{4'}$ is $-OCO-(CH_2)_{p6}-CO-$, $-NHCO-(CH_2)_{p7}-CO-$, or $-L^{4a}-(CH_2)_{q8}-CO-$where p6, p7, and q8 are each independently an integer of from 1 to 5, and $L^{4a}$ is OCONH, $-CH_2NHCO-$, NHCOO, NHCONH, CONH, or COO;
$R^{9'}$ and $R^{10'}$ are each independently a hydrogen atom, or an unsubstituted or substituted linear or branched alkyl

group having 1 to 12 carbon atoms; and k' represents an integer of from 30 to 20,000.

In the above-mentioned formulae (III) and (IV), an alkyl moiety in the linear or branched alkyloxy group, alkyl-substituted imino group, and alkyl group each having 1 to 12 carbon atoms, which are defined by the $R^{1'}$ and $R^{2'}$ groups, may be, for example, a methyl group, an ethyl group, a n-propyl group, an isopropyl group, an-butyl group, a sec-butyl group, a tert-butyl group, a n-hexyl group, a decyl group, or an undecyl group. An alkenyl or alkynyl moiety in the linear or branched alkenyloxy group having 2 to 12 carbon atoms, or the linear or branched alkynyloxy group having 2 to 12 carbon atoms may be exemplified by an alkenyl or alkynyl moiety including a double bond or a triple bond in the alkyl group having 2 or more carbon atoms as exemplified above.

For such group or moiety, the substituent(s) in a "substituted" case may be exemplified by, but not limited to, a $C_{1-6}$ alkoxy group, an aryloxy group, an aryl $C_{1-3}$ oxy group, a cyano group, a carboxyl group, an amino group, a $C_{1-6}$ alkoxycarbonyl group, a $C_{2-7}$ acylamide group, a tri-$C_{1-6}$ alkyl siloxy group, a siloxy group, or a silylamino group, or may be exemplified by an acetalized formyl group, a f ormyl group, or a halogen atom (such as chlorine or fluorine) . In this context, for example, the expression "$C_{1-6}$" means 1 to 6 carbon atoms and is used with the same meaning in the following description. In addition, an unsubstituted or substituted linear or branched alkyl moiety having 1 to 12 carbon atoms in the unsubstituted or substituted linear or branched alkylcarbonyl group having 1 to 24 carbon atoms may be selected with reference to the examples, and an alkyl moiety having 13 or more carbon atoms may be, for example, a tridecyl group, a tetradecyl group, a pentadecyl group, a nonadecyl group, a docosanyl group, or a tetracosyl group.

The binding order of the repeating units having the $R^{3a}$, $R^{3b}$, $R^{4a}$, and $R^{4b}$ groups is arbitrary, and it may be a random structure, or it may be a block structure. When both of the $R^{3a}$ and $R^{3b}$ groups represent an ethylene group, typically polyamino acids are represented in which n' represents an integer of 0 or a polyamino acid inwhichm' -n' represents an integer of 0. The former represents, for example, poly-$\alpha$-glutamic acid, which is obtained by the polymerization of an N-carboxylic anhydride of glutamic acid $\gamma$-benzyl ester, and the latter represents, for example, poly-$\gamma$-glutamic acid that strains of the genus Bacillus bacteria, such as *Bacillus natto,* produce. On the other hand, when both the $R^{3a}$ and $R^{3b}$ groups represent a methylene group, it is understood that the respective repeating units having those groups may coexist with each other. The same holds true for the $R^{4a}$ and $R^{4b}$ groups. It is preferred that the $R^{3a}$ and $R^{3b}$ groups each represent an ethylene group, and the $R^{4a}$ and $R^{4b}$ groups each represent a methylene group from the viewpoint of production efficiency.

In case the aliphatic hydrocarbon group is saturated in the definition of the $R^{6a}$ and $R^{6b}$ groups, the saturated aliphatic hydrocarbon group is equivalent to an alkyl group having 6 to 27 carbon atoms. Examples of the alkyl group include a hexyl group (such as a n-hexyl group), a decyl group, an undecyl group, a dodecyl group, a tridecyl group, a tetradecyl group, a pentadecyl group, ahexadecyl group, aheptadecylgroup, anoctadecyl group, anonadecyl group, an icosyl group, an eicosyl group, a henicosyl group, a heneicosyl group, a docosyl group, a tricosyl group, a tetracosyl group, a pentacosyl group, a hexacosyl group, and a heptacosyl group. In case the aliphatic hydrocarbon group is unsaturated in the definition of the $R^{6a}$ and $R^{6b}$ groups, examples of the unsaturated aliphatic hydrocarbon group include groups each obtained by changing 1 to 5 carbon-carbon single bonds in the chain of the alkyl group exemplified above to carbon-carbon double bonds.

Examples of the aromatic hydrocarbon group having 6 to 27 carbon atoms in the definition of the $R^{6a}$ and $R^{6b}$ groups include an aryl group and an aralkyl group. Preferred specific examples of those groups include a phenyl group, a naphthyl group, a tolyl group, a xylyl group, a benzyl group, and a phenethyl group.

The sterol from which the steryl group in the definition of the $R^{6a}$ and $R^{6b}$ groups results means a natural, semisynthetic, or synthetic compound based on a cyclopentanone hydrophenanthrene ring ($C_{17}H_{28}$), and means derivatives thereof as well. Examples of the natural sterol may include, but are not limited to, cholesterol, cholestanol, dihydrocholesterol, cholic acid, campesterol, and sitosterol. Examples of the semisynthetic or synthetic sterol may include synthetic precursors of the natural sterols (encompassing compounds in which as necessary, if present, part or all of predetermined functional groups and hydroxy groups are protected with hydroxy-protecting groups known in the art, or carboxyl groups are protected by carboxyl protection). In addition, a $C_{1-12}$ alkyl group and/or a halogen atom (such as chlorine, bromine, or fluorine) may be introduced into the cyclopentanone hydrophenanthrene ring of the sterol derivative as long as the obj ect of the present invention is not adversely affected. The ring system may be saturated, or maybe partially unsaturated. The sterol fromwhich the steryl group results is preferably a sterol originating from animal or vegetable oil such as cholesterol, cholestanol, dihydrocholesterol, cholic acid, campesterol, or sitosterol, more preferably cholesterol, cholestanol, or dihydroxycholesterol, particularly preferably cholesterol.

In the $R^{7a}$ and $R^{7b}$ groups, the groups selected from the following groups are defined:

$-NH-(CH_2)_{p1}-[NH-(CH_2)q_1-]r_1NH_2$ (i) ;

$-NH-(CH_2)_{p2}-N[-(CH_2)_{q2}-NH_2]_2$ (ii) ;

$-NH-(CH_2)_{p3}-N\{[-(CH_2)_{q3}-NH_2]\ [-(CH_2)_{q4}-NH-]_{r2}H\}$ (iii) ;

and

$-NH-\ (CH_2)_{p4}-N\{-(CH_2)_{q5}-N[-(CH_2)_{q6}-NH_2]_2\}_2$ (iv) ;

preferably they are identical groups, and more preferably they are the group represented by formula (i). In addition, p1 to p4 and q1 to q6 are each independently preferably 2 or 3, more preferably 2. On the other hand, it is preferred that r1 and r2 are each independently an integer of from 1 to 3.

m' -n' and n' each represent the number of repetitions of hydrophobic amino acid residues, and x'-y', y', and z' each represent the number of repetitions of cationic amino acid residues. The percentage of the number of repetitions (x'+z') of cationic amino acid residues with respect to the total number of repetitions (m'+x'+z') of amino acid residues is, for example, 20% or more, preferably 35% or more. The percentage is, for example, 80% or less, preferably 60% or less, morepref erably 50% or less. x' is preferably from 1 to 20, more preferably from 1 to 15, still more preferably from 1 to 10, particularly preferably from 1 to 5. When x' is 1 or more, the polyamino acid of the present invention includes at least the $R^{7a}$ group or the $R^{7b}$ group. When taken up into an endosome (pH 5.5) and exposed to a lower pH, the backbone polymer unit $\alpha$ having such structure undergoes further protonation of the cationic polyamino acid and exerts a buffer effect (or proton sponge effect), and thus the endosomal escape of the drug to be delivered can be facilitated by the polymer micelle pharmaceutical composition being simultaneously taken up by endocytosis.

The binding order of the respective repeating units within the polyamino acid segment of the backbone polymer unit $\alpha$ represented by formulae (III) and (IV) is arbitrary, and it may be a random structure, or it may be a block structure. When the polyamino acid segment is a block type containing the segment formed of the cationic amino acid residues and the segment formed of the hydrophobic amino acid residues, large functional unit groups in which functional units capable of inducing endocytosis and functional units capable of inducing endosomal escape are grouped, respectively, are formed, and hence the functions can be more certainly induced.

The above-mentioned $L^{1'}$ and $L^{3'}$ are each independently -S-S- or a valence bond. On the other hand, $L^{2'}$ is -NH-, -O-, $-O(CH_2)_{p5}-NH-$, or $-L^{2a}-(CH_2)_{q7}-L^{2b}-$ where p5 and q7 are each independently an integer of from 1 to 5, $L^{2a}$ is OCO, OCONH, NHCO, NHCOO, NHCONH, CONH, or COO, and $L^{2b}$ is NH or O. In addition, $L^{4'}$ is $-OCO-(CH_{2)p6}-CO-$, $-NHCO-(CH_2)_{p7}-CO-$, or $-L^{4a}-(CH_2)_{q8}-CO-$ where p6 , p7, and q8 are each independently an integer of from 1 to 5, and $L^{4a}$ is OCONH, $-CH_2NHCO-$, NHCOO, NHCONH, CONH, or COO. In the definition, the combination of $L^{1'}$ and $L^{2'}$, and the combination of $L^{3'}$ and $L^{4'}$ each need to be such that the groups may together form one linking group. When, for example, $L^{2'}$ is -NH-, $L^{1'}$ is not -S-S-but rather is a valence bond. A combination that forms a linking group in case $L^{1'}$ or $L^{3'}$ is -S-S- is preferred as the combination.

Examples of the linear or branched alkyl group having 1 to 12 carbon atoms in the definition of the $R^{9'}$ and $R^{10'}$ groups include the same groups as the alkyl moieties of the linear or branched alkyloxy group, alkyl-substituted imino group, and alkyl group each having 1 to 12 carbon atoms in the definition of the $R^{1'}$ and $R^{2'}$ groups. In addition, the same applies to its substituent.

k', which represents the number of repetitions of ethylene glycol (or oxyethylene), represents an integer of, for example, from 30 to 20,000, preferably from 40 to 2,000, still more preferably from 50 to 1,000.

The backbone polymer unit $\alpha$ may be formed by, for example, coupling the cationic polyamino acid and a hydrophilic polymer by a known method as they are, or as necessary, after purification to narrow their molecular weight distributions. In addition, for example, the block copolymer of the general formula (III) may be produced by: performing anionic living polymerization through the use of an initiator capable of providing $R^{9'}$ to form a polyethylene glycol chain; then introducing an amino group to the growing end side; polymerizing, from the resultant amino end, an N-carboxylic anhydride (NCA) of a protected amino acid, such as $\beta$-benzyl-L-aspartate, $\gamma$-benzyl-L-glutamate, or N$\varepsilon$-Z-L-lysine; and introducing a cationic group into a side chain of the resultant polyamino acid. It should be noted that a structural change due to nucleophilic attack by polyamine (such as the formation of an imide ring through the dealcoholization of an amino acid ester residue) may occur in some amino acid ester residues during the process of the synthesis of the cationic polyamino acid, and a block copolymer containing a residue which has undergone such structural change is herein also regarded as being included in the general formulae (III) and (IV). In addition, some NH groups and $NH_2$ groups in the cationic amino acid residues may be converted into salts (mainly hydrochlorides) owing to the use of an acid (mainly hydrochloric acid) in the synthesis process, and a block copolymer containing such structure is herein also regarded as being included in the general formulae (III) and (IV).

The polyamino acid chain segment of the backbone polymer unit $\alpha$ represented by the general formulae (III) and (IV) may be produced by, for example: polymerizing an N-carboxylic anhydride (NCA) of a protected amino acid known per se, such as $\beta$-benzyl-L-aspartate, Y-benzyl-L-glutamate, or N$\varepsilon$-Z-L-lysine to produce a polyamino acid ester; and then performing aminolysis using a polyamine corresponding to the $R^{7a}$, $R^{7b}$, and $R^{8'}$ groups to introduce cationic groups into side chains of polyamino acid.

In one embodiment, γ-benzyl-L-glutamate is polymerized and then β-benzyl-L-aspartate is polymerized, followed by a reaction with an amine compound, such as diethylenetriamine (DET). Thus, poly(β-benzyl-L-aspartate) is preferentially subjected to an ester-amide exchange reaction, with the result that an amine residue, such as a DET group, is introduced into an aspartic acid side chain. As a result, there may be obtained block-type cationic polyamino acid formed of an aspartic acid-derived cationic amino acid residue segment having a cationic group introduced into a side chain and a glutamic acid-derived hydrophobic amino acid residue segment having a benzyl group introduced into a side chain. On the other hand, when β-benzyl-L-aspartate and γ-benzyl-L-glutamate are simultaneously polymerized, followed by a reaction with an amine compound, such as diethylenetriamine (DET), there may be obtained random-type cationic polyamino acid in which an aspartic acid-derived cationic amino acid residue having a cationic group introduced into a side chain and a glutamic acid-derived hydrophobic amino acid residue having a benzyl group introduced into a side chain are arbitrarily arranged.

It should be noted that a structural change due to nucleophilic attack by an amine (such as the formation of an imide ring through the dealcoholization of an amino acid ester residue) may occur in some amino acid ester residues during the synthesis process, and block copolymers containing (a) residue(s) which has (have) undergone such structural change are herein also regarded as being included in the general formulae (III) and (IV). In this case, the number of residues which have undergone the structural change is not included in either of the number of cationic polyamino acid residues or the number of hydrophobic amino acid residues. In addition, some NH groups and $NH_2$ groups in the cationic amino acid residues may be converted into salts (mainly hydrochlorides) owing to the use of an acid (mainly hydrochloric acid) in the synthesis process, and block copolymers containing (a) residue (s) which has (have) undergone such structural change are herein also regarded as being included in the general formulae (III) and (IV). That is, some NH groups and $NH_2$ groups in the $R^{7a}$, $R^{7b}$, and $R^{8'}$ groups may be converted into salts (such as hydrochlorides).

[3. Backbone Polymer Unit β]

The backbone polymer unit β has the hydrophilic polymer chain segment and the hydrophobic polymer chain segment, and is radially arranged in the state in which the hydrophilic polymer chain segment is directed outward and the hydrophobic polymer chain segment is directed inward. The polymer micelle pharmaceutical composition may contain two or more kinds of the backbone polymer unit β.

The backbone polymer unit β may have a drug bound to the hydrophobic polymer chain segment.

The backbone polymer unit β desirably has a higher hydrophobicity than that of the backbone polymer unit α. The higher hydrophobicity may result from the drug. For example, the backbone polymer unit β, even while having more hydrophilic groups than the backbone polymer unit α, may have a higher hydrophobicity than the backbone polymer unit α owing to the drug.

The backbone polymer unit β may be a complex of a drug and a block copolymer, the complex being represented by the general formula: $A_2$-$B_2$(-D). $A_2$ represents a polyethylene glycol chain segment, $B_2$ represents a polyamino acid chain segment, and D represents the drug.

Examples of the polyethylene glycol chain segment and the polyamino acid chain segment of the backbone polymer unit β may include similar ones to those in the case of the backbone polymer unit α. It should be noted that the polyamino acid chain segment of the backbone polymer unit β does not need to have any hydrophilic group in a side chain.

The backbone polymer unit β does not exclude the state of having a target binding site, but is typically in the state of containing a drug but being free of a target binding site. When the polymer micelle pharmaceutical composition is in the state in which the drug and the target binding site are contained in different polymer units, in case the micelle structure has collapsed before the polymer micelle pharmaceutical composition migrates within the bloodstream to the vicinity of the target cell, the drug can be discharged out of the body through metabolism, and hence the occurrence of side effects is easily avoided. Further, it is not necessary to bind both the compound having a target binding site and the drug to the same block copolymer, and hence the deactivation of the drug or the compound for target binding during synthesis is easily avoided. It should be noted that when the target binding site is loaded into the backbone polymer unit β, the target binding site is desirably bound to a projecting end of the hydrophilic polymer chain segment.

Preferred specific examples of the backbone polymer unit β include the block copolymers represented by the general formulae (I) and (II). It should be noted that with regard to $R^6$s in the general formulae (I) and (II), 10% or more of the total number (m+x) of $R^6$s is/are each a residue of a drug, which may have a linking group, and the remaining group(s), if present, is/are a hydrogen atom or a hydrophobic organic group. The percentage of the residues of the drug, which may have a linking group, with respect to the total number (m+x) is preferably from 10% to 100%, more preferably from 10% to 70%. $R^6$s are each independently selected in each amino acid unit in one block copolymer. The binding order of the respective repeating units within the polyamino acid segment of the backbone polymer unit β is arbitrary.

Examples of the drug may include nucleic acids (such as a nucleoside, a DNA, a RNA, a siRNA, and a microRNA), a nucleic acid derivative, a vaccine, an antibody having pharmacological activity (the so-called antibody medicine), docetaxel, a camptothecin, epothilone A, epothilone B, epothilone C, epothilone D, and derivatives of these epothilones,

temsirolimus, everolimus, trabectedin, vorinostat, octreotide acetate, mitoxantrone, vincristine, cephalexin, cefaclor, ampicillin, bacampicillin, amoxicillin, kanamycin, amikacin, arbekacin, dibekacin, sisomicin, tobramycin, erythromycin, clarithromycin, rokitamycin, chloramphenicol, vancomycin, fluconazole, vidarabine, acyclovir, didanosine, zidovudine, zalcitabine, lamivudine, zanamivir, oseltamivir, lopinavir, andritonavir. Examples of the derivatives of the epothilones may includepatupilone, ixabepilone, BMS-310705, KOS-862, and ZK-EPO.

Examples of the nucleic acid derivative may include gemcitabine, nelarabine, clofarabine, decitabine, streptozocin, doxifluridine, and fludarabine. The nucleic acid may be bound to the backbone polymer unit β, for example, through a covalent bond and/or an electrostatic bond. The nucleic acid derivative may be a salt, but when the nucleic acid derivative is bound to the backbone polymer unit β through an ester bond, it is preferred that the nucleic acid derivative is not a salt.

The drug and the block copolymer unit may be bound, for example, through a covalent bond and/or an electrostatic bond. Examples of the covalent bond include a single bond and a divalent linking group. An example of the divalent linking group is a divalent linking group which has 0 to 5 carbon atoms and may contain an amide bond, an ester bond, an ether bond, and/or a hydrazide bond. The divalent linking group is preferably an ester bond, an amide bond, or a hydrazide bond.

As aspects of the binding, one of the drugs and one block copolymer unit may be bound through one covalent bond or electrostatic bond, or may be bound through two or more covalent bonds and/or electrostatic bonds. Further, one of the drugs and two or more block copolymer units may be bound through two or more covalent bonds and/or electrostatic bonds (that is, two or more block copolymer units are bound in a state of being cross-linked through one drug).

When the drug has a plurality of hydroxy groups, the backbone polymer unit β may have a structure in which one or more of the hydroxy groups are ester-bonded to carboxyl groups of a polyamino acid side chain. Herein, a structure in which one drug is ester-bonded to a plurality of carboxyl groups in the polyamino acid side chain, and a structure in which two or more block copolymer moieties are cross-linked through one drug are also regarded as being included in the backbone polymer unit β.

Examples of the ester bond include: an ester bond formed through a reaction between a drug having a hydroxy group, and a block copolymer unit having a carboxyl group; and an ester bond formed through a reaction between a drug having a carboxyl group, and a block copolymer unit having a hydroxy group.

Examples of the amide bond include: an amide bond formed through a reaction between a drug as an amine, and a block copolymer unit having an ester group; and an amide bond formed through a reaction between a drug having an amino group, and a block copolymer unit having a carboxyl group.

An example of the hydrazide bond is a hydrazide bond formed by the binding of a drug having a ketone structure to a hydraz ide group of a block copolymer unit.

[4. Backbone Polymer Unit y]

The polymer micelle pharmaceutical composition may further include a block copolymer unit γ (backbone polymer unit γ) having a hydrophilic polymer chain segment having a target binding site bound thereto, and a hydrophobic polymer chain segment, and being radially arranged together with the block copolymer units α and β in the state in which the hydrophilic polymer chain segment is directed outward and the hydrophobic polymer chain segment is directed inward. The backbone polymer unit γ may be arranged in the state in which the target binding site is directed outward.

The backbone polymer unit γ desirably has such a degree of hydrophobicity (hardness) that the particle backbone of the polymer micelle can be formed; more specifically, it desirably has a degree of hydrophobicity (hydrophobic structure) of almost the same amount as the backbone polymer unit (3.

Examples of the hydrophilic polymer chain segment and the hydrophobic polymer chain segment of the backbone polymer unit γ may include similar ones to the respective segments in the case of the backbone polymer unit α. It should be noted that the hydrophobic polymer chain segment of the backbone polymer unit γ does not need to have any hydrophilic group in a side chain.

The backbone polymer unit γ may be a complex of a compound having a target binding site and a block copolymer, the complex being represented by the general formula: $Z\text{-}A_3\text{-}B_3$. Z represents the compound having a target binding site, $A_3$ represents a polyethylene glycol chain segment, and $B_3$ represents a polyamino acid chain segment.

An example of the compound having a target binding site may be, as described above, a protein, a peptide, or a sugar chain which forms a binding pair with a substance of biological and viral origin. Examples of such protein may include: an antibody and a fragment thereof which bind to the substance of biological and viral origin; transferrin; and epidermal growth factor (EGF). Examples of the antibody may include antibodies capable of recognizing antigens including receptors and cell surface antigens, such as EGFR, Her2, CD20, VEGFR, and CD52, highly expressed on surfaces of medication targets typified by cancer cells. The antibody may be a monoclonal antibody, or may be a polyclonal antibody. The fragment of the antibody only needs to have a length which allows specific recognition of an antigen, and examples thereof may include (Fab')2 and Fab. Examples of the peptide may include insulin, LHRH, IGF,

and derivatives thereof. Examples of the sugar may include sugars having glucose, mannose, galactose, and fucose residues. The compound having a target binding site may be a compound capable of exhibitingpharmacological activity in itself (such as an antibody drug or a vaccine).

When a target with which the compound having a target binding site is to form a binding pair is a substance of viral origin, cells to which the substance is to be supplied are in a state in which cell death has occurred through the disruption of the cell membrane by a virus infecting the cells, and hence the polymer micelle pharmaceutical composition cannot be taken up into the cells through endocytosis. Therefore, herein, when the target is the substance of viral origin, cells present in proximity of the target are regarded as cells to which the target is to be supplied. When the substance of viral origin is present extracellularly, it is highly probable that such cells in the surroundings are infected with the virus, and hence the supply of the drug to the surrounding cells is of significance.

Examples of the polyethylene glycol chain segment and the polyamino acid chain segment of the backbone polymer unit $\gamma$ may include similar ones to those in the cases of the backbone polymer units $\alpha$ and $\beta$, and the same applies to the formation method therefor.

Preferred specific examples of the backbone polymer unit $\gamma$ include the block copolymers represented by the general formulae (I) and (II). The polymer micelle pharmaceutical composition of the present invention may contain two or more kinds of the backbone polymer unit $\gamma$. It shouldbe noted that in the general formulae (I) and (II), $R^1$ and $R^3$ each represent a compound having a target binding site. Further, $R^6$s each represent a hydrogen atom or a hydrophobic organic group, and the percentage of the hydrogen atoms with respect to the total number (m+x) is, for example, from 0% to 60%, preferably from 0% to 20%, and the remaining group(s) is (are) a hydrophobic organic group.

The backbone polymer unit $\gamma$ may be formed by a condensation or addition reaction of a block copolymer having, in the $\alpha$-end of its polyethylene glycol chain segment, a linking group such as a hydroxyl group, a carboxyl group, an aldehyde group, an amino group, a mercapto group, or a maleimide group, and the compound having a target binding site.

[5. Production Method for Polymer Micelle Pharmaceutical Composition]

The polymer micelle pharmaceutical composition of the present invention may be formed by, for example: dissolving the backbone polymer unit $\alpha$ and the backbone polymer unit $\beta$, or the backbone polymer unit $\alpha$, the backbone polymer unit $\beta$, and the backbone polymer unit $\gamma$ in an organic solvent; mixing the contents to achieve homogenization; subjecting the resultant solution to evaporation under reduced pressure; adding water to the resultant film of polymers; and mixing the contents to allow the polymers to self-assemble into a micellar form. In addition, for example, the polymer micelle pharmaceutical composition of the present invention may be formed by mixing those backbone polymer units in an aqueous solution to allow the backbone polymer units to self-assemble into a micellar form. Further, for example, the polymer micelle pharmaceutical composition of the present invention may also be formed by: dissolving the backbone polymer unit $\alpha$, the backbone polymer unit $\beta$, and a precursor of the backbone polymer unit $\gamma$ in an organic solvent; mixing the contents to achieve homogenization; subjecting the resultant solution to evaporation under reduced pressure; adding water to the resultant film of polymers; mixing the contents to allow the polymers to self-assemble into a micellar form; and then binding the compound having a target binding site to the $\alpha$-end of the hydrophilic segment of the precursor of the backbone polymer unit $\gamma$ to produce the backbone polymer unit $\gamma$. Further, for example, the polymer micelle pharmaceutical composition of the present invention may also be formed by: mixing those backbone polymer units in an aqueous solution to allow the backbone polymer units to self-assemble into a micellar form; and then binding the compound having a target binding site to the $\alpha$-end of the hydrophilic segment of the precursor of the backbone polymer unit $\gamma$ to produce the backbone polymer unity. Examples of the organic solvent include methanol and acetone. The aqueous solution may be formed by, for example, adding a water-miscible organic solvent, such as ethanol or dimethyl sulfoxide, and a known buffer to purified water.

Examples

Hereinafter, the present invention is more specifically described by way of Examples. The present invention is by no means limited by these examples.

(Example 1)

A Herceptin-bound docetaxel (DTX) micelle, which is a polymer micelle formulation having HERCEPTIN as the compound with a target binding site and encapsulating docetaxel (DTX) as the drug, was formed as described below.

(Backbone Polymer Unit α)

A polyethylene glycol-polyglutamic acid benzyl ester copolymer (one end of polyglutamic acid is acetylated; the average molecular weight (Da) of the PEG is 10,000; the average number of glutamic acid residues is 40; 60% of the hydrogen atoms of the carboxylic acids in the side chains are substituted with phenyl groups. The copolymer is hereinafter sometimes referred to as "PEG-PBLG (OBn: 60%)".) was used as the backbone polymer unit α.

(Backbone Polymer Unit β)

PEG-pAsp-DTX as the backbone polymer unit β was produced as described below. 500 mg of a polyethylene glycol-polyaspartic acid block copolymer (PEG-pAsp-Ac) in a state of having one end of polyaspartic acid acetylated was dissolved in 10 mL of anhydrous DMF (Kanto Chemical Co., Inc.), and then 1.06 g of docetaxel (ScinoPharm Taiwan Ltd.) was further added. It should be noted that the PEG-pAsp-Ac has an average molecular weight (Da) of the PEG of 10,000, has an average number of the aspartic acid residues of 40, and has carboxylic acid as the side chain of the aspartic acids. Subsequently, 160 mg of 4-dimethylaminopyridine (Wako Pure Chemical Industries, Ltd.) and 210 μL of N,N'-diisopropylcarbodiimide (Kokusan Chemical Co., Ltd.) were added in the stated order, and the mixture was stirred at room temperature overnight. The thus-obtained reaction liquid was added dropwise to 500 mL of a mixed solution of hexane and ethyl acetate (volume ratio: 1:1) to crystallize a polymer, and then the polymer was collected by filtration under reduced pressure. The polymer collected by filtration was suspended in 100 mL of purified water to form a polymer micelle, followed by ultrafiltration (Labscale TFF System manufactured by Millipore Corporation, molecular weight cutoff value: 100,000, 5-fold dilution and then concentration to 100 mL) . This ultrafiltration operation was repeated five times, followed by lyophilization. The polymer obtained by the lyophilization was added dropwise, in a state of being dissolved in 10 mL of anhydrous DMF, to 500 mL of a mixed solution of hexane and ethyl acetate (volume ratio: 1:1) to crystallize the polymer, and then the polymer was collected by filtration under reduced pressure. The polymer collected by filtration was washed by being added, while in a powder state, to 100 mL of a mixed solution of hexane and ethyl acetate (volume ratio: 1:1), and was then collected by filtration under reduced pressure. The polymer collected by filtration was dried under reduced pressure at room temperature overnight to provide 530 mg of PEG-pAsp-DTX as a pale yellow powder.

1 mg of PEG-pAsp-DTX was dissolved in 10 mL of a mixed solution of purified water and ethanol (volume ratio: 1:1), and its docetaxel content was measured based on absorbance with respect to light having a wavelength of 233 nm and was found to be 14.3 molecules per polymer. The PEG-pAsp-DTX is in the state in which docetaxel is bound to PEG-pAsp-Ac through an ester bond. The chain length of the PEG is 10 kDa.

(Maleimide-bound Polymer)

A maleimide-polyethylene glycol-polyglutamic acid copolymer having a maleimide group at a PEG end (one end of polyglutamic acid is acetylated; the average molecular weight (Da) of the PEG is 10,000; the average number of glutamic acid residues is 40; and all hydrogen atoms of the carboxylic acids in the side chains are substituted with phenyl groups.) was prepared. The copolymer is hereinafter sometimes referred to as "Maleimide-PEG-PBLG".

(Formation of Micelle)

PEG-PBLG (OBn: 60%), PEG-pAsp-DTX, and Maleimide-PEG-PBLG were precisely weighed at a weight ratio of 4:5:1 in separate vials, and were each dissolved by the addition of methanol or acetone. The respective solutions were mixed together, and then the solvent was evaporated with a rotary evaporator (manufactured by Büchi, ROTAVAPOR R-205, Vac(R) V-513) to form a film of mixed polymers, which was further dried for one day and night. 100 mM PBS (pH 7.4) was added to disperse the film, and the resultant was subjected to high-pressure dispersion treatment using a NanoVater (manufactured by Yoshida Kikai Co., Ltd., NM2-L200) to provide a micelle. The micelle fraction contains a micelle in which PEG-PBLG (OBn: 60%), PEG-pAsp-DTX, and Maleimide-PEG-PBLG are radially arranged.

(Reaction between HERCEPTIN and Maleimide)

Purified water was added to a vial containing HERCEPTIN at a molar ratio of 0.4 with respect to Maleimide-PEG-PBLG to prepare a Herceptin solution. The Herceptin solution was adjusted to have a final concentration of borate buffer of 50 mM and a final concentration of EDTA of 1 mM, and 10 mg/mL Traut' s Reagent (Pierce Biotechnology Inc.) was further added. After that, the mixture was left to stand still at 30°C for 45 minutes. Subsequently, the thus-obtained reaction liquid was purified by gel filtration [PD-10 manufactured by GE Healthcare Life Sciences, eluent: 100 mM sodium phosphate buffer (pH7.4), 1 mM EDTA], and a high-molecular-weight fraction was collected.

The collected liquid and the micelle fraction were mixed, and the mixture was left to stand still at 30°C for 2

hours to subject the maleimide group of Maleimide-PEG-PBLG and HERCEPTIN to reaction. Thus, a micelle containing a Herceptin-bound polymer as the backbone polymer unit γ was formed. The thus-obtained reaction liquid was purified by ultrafiltration (manufactured by Millipore Corporation, AMICON Ultra-15, membrane fraction: 300,000) to remove unreacted HERCEPTIN. Thus, a solution containing a Herceptin-bound docetaxel micelle having PEG-PBLG (OBn: 60%) was obtained. The docetaxel content in the micelle solution was 2.154 mg/mL.

(Comparative Example 1)

A solution containing a Herceptin-bound docetaxel micelle was obtained in the same manner as in Example 1 except that PEG-pAsp-DTX and Maleimide-PEG-PBLG were used at a weight ratio of 9 :1, and PEG-PBLG (OBn: 60%) was not used. The docetaxel content in the micelle solution was 1.273 mg/mL.

(Comparative Example 2)

A 10% sucrose solution containing a docetaxel micelle was obtained by using only PEG-pAsp-DTX. The docetaxel content in the micelle solution was 3.486 mg/mL.

[Cytotoxicity Test 1]

The cytotoxicity of the Herceptin-bound docetaxel micelle having PEG-PBLG (OBn: 60%) of Example 1 was compared to the cytotoxicity of each of the Herceptin-bound docetaxel micelle of Comparative Example 1 and the docetaxel micelle of Comparative Example 2. The cytotoxicity of each of the samples was evaluated as described below based on the WST method using human prostate cancer-derived PC-3 cells (HER2-negative) purchased from ATCC through Summit Pharmaceuticals International Corporation.

The PC-3 cells were seeded in a 96-well plate in a state of being suspended in 90 μL of a medium so that about 5,000 of the cells were contained per well, and the cells were cultured under a 5% $CO_2$ atmosphere at 37°C overnight. RPMI 1640 (Gibco™, Invitrogen) and 10% FBS (biowest) were used as the medium. Subsequently, a solution prepared by diluting any one of the samples with the medium so as to have any of various docetaxel concentrations was added to each well (10 μL per well), and culturing was performed under a 5% $CO_2$ atmosphere at 37°C for 72 hours. After that, WST Reagent (Dojindo Laboratories) was added (10 μL per well), and culturing was continued under a 5% $CO_2$ atmosphere at 37°C for about 2 hours. Absorbance with respect to light having a wavelength of 450 nm (Abs450) was measured for each well, and the cell growth rate (% Cell Growth) was calculated on the basis of the following equation. It should be noted that "Abs450 value of control" in the equationmeans the absorbance obtained fromawell in which culturing as described above was performed using a culture solution containing no docetaxel.

$$\%\ \text{cell growth} = \frac{\left(\text{Abs450 value after addition of sample liquid}\right) - \left(\text{Abs450 value of blank}\right)}{\left(\text{Abs450 value of control}\right) - \left(\text{Abs450 value of blank}\right)} \times 100$$

The docetaxel concentration was set to 0.05 ng/mL, 0.14 ng/mL, 0.41 ng/mL, 1.23 ng/mL, 3.7 ng/mL, 11.1 ng/mL, 33.3 ng/mL, or 100 ng/mL. The results were subjected to logarithmic approximation by placing the cell growth rate (%) on the vertical axis and the docetaxel concentration (ng/mL) on the horizontal axis, to calculate an IC25 (25% inhibition concentration (ng/mL)). The results are shown in Table 1.

It should be noted that specific examples of data on Cytotoxicity Test 1 are described below.

(In Case of Concentration in Terms of Docetaxel of 3.7 ng/mL)

The cell growth rate of the PC-3 cells (%) was 81.9% (Example 1), 106% (Comparative Example 1), or 109% (Comparative Example 2).

(In Case of Concentration in Terms of Docetaxel of 11.1 ng/mL)

The cell growth rate of the PC-3 cells (%) was 46.5% (Example 1), 59.6% (Comparative Example 1), or 62.4% (Comparative Example 2).

(In Case of Concentration in Terms of Docetaxel of 33.3 ng/mL)

The cell growth rate of the PC-3 cells (%) was 24.6% (Example 1), 30.0% (Comparative Example 1), or 33.9% (Comparative Example 2).

[Cytotoxicity Test 2]

TheIC25 (25% inhibition concentration (ng/mL)) was calculated by performing the same experiment as Cytotoxicity Test 1 except that: human stomach cancer-derived NCI-N87 cells (HER2-positive) purchased from ATCC through Summit Pharmaceuticals International Corporation were used; the number of NCI-N87 cells was set to about 10,000 cells per well; the docetaxel concentration was set to 0.5 ng/mL, 1.4 ng/mL, 4.1 ng/mL, 12.4 ng/mL, 37.0 ng/mL, 111.1 ng/mL, 333.3 ng/mL, or 1,000.0 ng/mL; and the cells were cultured under a 5% $CO_2$ atmosphere at 37°C for 1 hour, the medium was then removed from the wells, the wells were washed with phosphate buffered saline (PBS) twice, a fresh medium was then added (100 μL per well), and culturingwas continued until the total culture time became 72 hours. The results are shown in Table 1.

It should be noted that specific examples of data on Cytotoxicity Test 2 are described below.

(In Case of Concentration in Terms of Docetaxel of 37.0 ng/mL)

The cell growth rate of the NCI-N87 cells (%) was 82. 6% (Example 1) or 92.9% (Comparative Example 1).

(In Case of Concentration in Terms of Docetaxel of 111.1 ng/mL)

The cell growth rate of the NCI-N87 cells (%) was 73.0% (Example 1) or 88.0% (Comparative Example 1).

[Table 1]

| | Example 1 | Comparative Example 1 | Comparative Example 2 |
|---|---|---|---|
| PC-3 cells | 3.3 | 8.4 | 9.2 |
| NCI-N87 cells | 88.1 | 403.4 | N.D. |

The results revealed that the polymer micelle pharmaceutical composition having PEG-PBLG (OBn: 60%) (Example 1) exhibited a cytocidal effect in a much lower concentration range as compared to the polymer micelle pharmaceutical compositions free of PEG-PBLG (OBn: 60%) (Comparative Examples 1 and 2). The results were observed irrespective of the presence or absence of an antigen for Herceptin in the cells. Accordingly, it is considered that the favorable effect based on the admixing of PEG-PBLG (OBn: 60%) is exhibited irrespective of the presence or absence of the backbone polymer unit γ having a target binding site. Further, because the maintenance/collapsing of the particle structure of the polymer micelle pharmaceutical composition is considered to be the essence of the mechanism of action, it is considered that there is no particular restriction on the drug to be encapsulated.

[Anti-tumor Effect Confirmation Test]

HER2-positive human stomach cancer NCI-N87 cells were purchased from ATCC through Summit Pharmaceuticals International Corporation. The cells were cultured using an RPMI 1640+10% FBS medium under 5% $CO_2$ at 37°C, and grown until the number of cells reached a number required for transplantation. The cells were suspended in saline to prepare a suspension having a concentration of $6.0\times10^7$ cells/mL. To the cell suspension, an equal amount of a substrate for cell culture (manufactured by Nippon BectonDickinson Company, Ltd., trade name: "BD Matrigel" (registered trademark)) cooled with ice was added (cell concentration: $3.0\times10^7$ cells/mL). The resultant was inoculated subcutaneously into the right abdomens of male nude mice (Balb nu/nu, 6-weeks old, made by Charles River Laboratories Japan, Inc.) at $3.0\times10^6$ cells/100 μL per mouse. After that, the nude mice were reared for 15 days, and a medicament was administered when the tumor volume reached about 130 $mm^3$. Themicelle of Example 1 and the micelle of Comparative Example 1 were each administered into the tail vein in a single dose of 7 mg/kg in terms of docetaxel. Further, as Comparative Example 3, a 10% sucrose solution was administered as the medium. In each of the Example and Comparative Examples, five animals were used as a group. Immediately before the administration of the micelle formulation and on day 10 after the administration, the tumor volume and the body weight of each of the nude mice were measured, and relative values of the tumor volume and the body weight on day 10 after the administration with respect to those immediately before the administration of the micelle formulation were measured. The results are shown in Table 2.

[Table 2]

| | Example 1 | Comparative Example 1 | Comparative Example 3 |
|---|---|---|---|
| Relative tumor volume | 119.6% | 147.5% | 174.8% |
| Relative body weight | 104.6% | 104.3% | 106.3% |

From the results shown in Table 2, it was found that, when the polymer micelle pharmaceutical composition having PEG-PBLG (OBn: 60%) (Example 1) was administered, while a state was achieved in which the changes in mouse body weight did not differ, the growth of the human stomach cancer NCI-N87 cells could be remarkably suppressed as compared to the case of administering the polymer micelle pharmaceutical composition free of PEG-PBLG (OBn: 60%) (Comparative Example 1) and the case of administering the medium (Comparative Example 3).

(Test Example 1)

(Preparation of Alexa 488-labeled SH-modified Herceptin)

1 mL of a Herceptin formulation (22 mg/mL) was dispensed into a 1.5-mL microtube. In order to remove histidine contained in the formulation, the formulation was purified by gel filtration (manufactured by GE Healthcare Japan, trade name: "PD-10"). The concentration of the antibody was measured using a spectrophotometer at 280 nm and adjusted to 10 mg/mL. An Alexa 488 labeling kit (manufactured by Life Technologies, trade name: "Alexa Fluor 488 Protein Labeling Kit") was used to label an amino group in the antibody in accordance with the protocol described in the attached document. Unreacted labeling reagent (fluorescein derivative) was removed using PD-10, and the concentration of the antibody was calculated again with the spectrophotometer (280 nm and 494 nm). 20 equivalents of a 10 mg/mL Traut's Reagent solution (1 mM EDTA borate buffer (pH 8.0)) was added with respect to 100 parts by weight of the Alexa 488-labeled Herceptin. An SH modification reaction with Traut's Reagent was performed in a water bath at 30°C for 45 minutes, and after the completion of the reaction, PD-10 was used to remove the excess of Traut's Reagent. The Alexa 488-labeled SH-modified Herceptin in this state is hereinafter denoted as "Alexa-Herceptin-SH".

(Formation of Micelle)

90 mg of PEG-PBLG (OBn: 100%) and 10 mg of Maleimide-PEG-PBLG (content: 9:1 in the stated order) were weighed, and each polymer was dissolved with methanol or acetone. The respective polymer solutions were mixed and homogenized in a 100-mL recovery flask, and the organic solvent was evaporated with an evaporator to provide an amorphous film of mixed polymers. 10 mL of PBS was added to about 100 mg of the film of the polymers obtained, and the contents were dissolved using an ultrasonic device. The solution was subjected to polymer micellization using a high-pressure disperser (manufactured by Yoshida Kikai Co., Ltd.: NanoVater NM2-L200) to provide a 10 mg/mL mixed polymer micelle solution. The micelle solution obtained by the high-pressure dispersion treatment was measured for its particle diameter with a dynamic light scattering photometer.

(Reaction between Alexa-Herceptin-SH and Maleimide)

0.75 mL of the Alexa-Herceptin-SH solution obtained in the foregoing (1.5 mg in terms of Herceptin) and 4 mL of the 10 mg/mL mixed polymer micelle solution were mixed using a CRYOVIAL, and subjected to a reaction at 30°C for 2 hours (addition reaction between maleimidein Maleimide-PEG-PBLG and SH-modified Herceptin). After the reaction, the resultant was stored at below 4°C overnight, and then a particle diameter measurement was performed. In order to remove unreacted Alexa-Herceptin-SH contained in the reaction liquid, the resultant was concentrated 5-fold three times at below 4°C and 3,000 rpm using a centrifugal ultrafiltration filter (manufactured by Merck Millipore Corporation, trade name: "AMICON Ultra ULTRACEL-100K: 100,000 MWCO"). To the supernatant after the centrifugal ultrafiltration, 5 equivalents of a 10 mg/mL L-cysteine PBS solution was added with respect to the stoichiometric amount of maleimide, and the mixture was subjected to reaction at room temperature for 30 minutes to quench the unreacted maleimide moiety in the micelle. In order to remove the excess of L-cysteine, the resultant was purified by gel filtration (manufactured by GE Healthcare Japan, trade name: "PD-10"). At this time, solution replacement was performed with a 10% sucrose aqueous solution (w/v) as the eluent to prevent micelle aggregation. The resultant antibody-bound micelle solution was sterilized by filtration (filter pore size: 0.22 μm) and subjected to a dispensing operation to complete the preparation of an Alexa 488-labeled Herceptin-bound micelle formulation. The particle diameter of the finished formulation was measured with a dynamic light scattering photometer. The micelle formulation corresponds to an aspect in which the content of the backbone polymer unit $\alpha$ is 0 mass%, the content of the backbone polymer unit $\beta$ is 90 mass%, and the content of the backbone polymer unit $\gamma$ is 10 mass%. It should be noted that this may be paraphrased as an aspect in which the content of apseudo unit $\alpha$ having a hydrophilic group-carrying percentage in the hydrophobic segment chain of 0% is 40 mass% and the content of the unit $\beta$ is 50 mass%.

(Test Example 2)

An Alexa 488-labeled Herceptin-bound micelle formulation was prepared in the same manner as in Test Example

1 except that 10 mg of PEG-PBLG (OBn: 76.8%), 80 mg of PEG-PBLG (OBn: 100%), and 10 mg of Maleimide-PEG-PBLG (content: 1:8:1 in the stated order) were used. The micelle formulation corresponds to an aspect in which the content of the backbone polymer unit α is 10 mass% and the hydrophilic group-carrying percentage of the hydrophobic segment chain in the unit α is 23.2%. In addition, the micelle formulation, like Test Example 4 below, corresponds to an aspect in which the content of the backbone polymer unit β is 80 mass% and the content of the backbone polymer unit γ is 10 mass%.

(Test Example 3)

An Alexa 488-labeled Herceptin-bound micelle formulation was prepared in the same manner as in Test Example 1 except that 40 mg of PEG-PBLG (OBn: 76.8%), 50 mg of PEG-PBLG (OBn: 100%), and 10 mg of Maleimide-PEG-PBLG (content: 4:5:1 in the stated order) were used. The micelle formulation corresponds to an aspect in which the content of the backbone polymer unit α is 40 mass% and the hydrophilic group-carrying percentage of the hydrophobic segment chain in the unit α is 23.2%. In addition, the micelle formulation, like Test Example 5 below, corresponds to an aspect in which the content of the backbone polymer unit β is 50 mass% and the content of the backbone polymer unit γ is 10 mass%.

(Test Example 4)

An Alexa 488-labeled Herceptin-bound micelle formulation was prepared in the same manner as in Test Example 1 except that 10 mg of PEG-PBLG (OBn: 60.6%), 80 mg of PEG-PBLG (OBn: 100%), and 10 mg of Maleimide-PEG-PBLG (content: 1:8:1 in the stated order) were used. The micelle formulation corresponds to an aspect in which the content of the backbone polymer unit α is 10 mass% and the hydrophilic group-carrying percentage of the hydrophobic segment chain in the unit α is 39.4%.

(Test Example 5)

An Alexa 488-labeled Herceptin-bound micelle formulation was prepared in the same manner as in Test Example 1 except that 40 mg of PEG-PBLG (OBn: 60.6%), 50 mg of PEG-PBLG (OBn: 100%), and 10 mg of Maleimide-PEG-PBLG (content: 4:5:1 in the stated order) were used. The micelle formulation corresponds to an aspect in which the content of the backbone polymer unit α is 40 mass% and the hydrophilic group-carrying percentage of the hydrophobic segment chain in the unit α is 39.4%.

(Test Example 6)

An Alexa 488-labeled Herceptin-bound micelle formulation was prepared in the same manner as in Test Example 1 except that 20 mg of PEG-PBLG (OBn: 60.6%), 70 mg of PEG-PBLG (OBn: 100%), and 10 mg of Maleimide-PEG-PBLG (content: 2:7:1 in the stated order) were used. The micelle formulation corresponds to an aspect in which the content of the backbone polymer unit α is 20 mass% and the hydrophilic group-carrying percentage of the hydrophobic segment chain in the unit α is 39.4%. In addition, the micelle formulation corresponds to an aspect in which the content of the backbone polymer unit β is 70 mass% and the content of the backbone polymer unit γ is 10 mass%.

(Cellular Uptake Test for Micelle Formulations)

NCI-N87 cells were added in a state of being suspended in 90 μL of a medium so that about 250,000 of the cells were contained per 1.5-mL microtube. RPMI 1640 (Gibco™, Invitrogen) and 10% FBS (biowest) were used as the medium. The micelle formulations of Test Examples 1 to 6 were each added to a tube so as to achieve a final concentration of 4 μg/mL in terms of HERCEPTIN, and culturing was performed under a 5% $CO_2$ environment at 37°C for 2 hours. The cells were washed with PBS twice, the cells were immobilized with 2% paraformaldehyde/PBS, and fluorescence intensity (FL1-A) was measured using a flow cytometer (manufactured by Nippon Becton DickinsonCompany, Ltd., tradename: "BDAccuri™C6 flow cytometer"). The resultant fluorescence intensity values were corrected to values with the polymer micelle concentration being adjusted to be constant, and the amount of each of the micelle formulations delivered to cell, more specifically, the total amount of the amount of the micelle formulation bound to the cell surface and the amount of the micelle formulation taken up into the cell, was evaluated. The results are shown in FIG. 2. It should be noted that the vertical axis of the graph of FIG. 2 is a relative value when the median value of the fluorescence intensity calculated in Test Example 1 is defined as 100%. As shown in FIG. 2, when the degree of hydrophilicity of the hydrophobic structure moiety in the micelle formulation resulting from the unit α is controlled to a predetermined range, the delivery properties of the micelle formulation to the target cell is more certainly enhanced.

Industrial Applicability

The present invention can be suitably utilized in the field of, for example, pharmaceutical formulations, such as anticancer agents.

## Claims

1. A polymer micelle pharmaceutical composition, comprising:

   a block copolymer unit α having a hydrophilic polymer chain segment and a hydrophobic polymer chain segment; and
   a block copolymer unit β having a hydrophilic polymer chain segment and a hydrophobic polymer chain segment, wherein:

      the block copolymer unit α and the block copolymer unit β are radially arranged in the state in which the hydrophilic polymer chain segments are directed outward and the hydrophobic polymer chain segments are directed inward; and
      the hydrophobic polymer chain segment of the block copolymer unit α is constituted of repeating units having side chains, at least one of the side chains having a hydrophilic group.

2. The polymer micelle pharmaceutical composition according to claim 1, wherein 20% to 80% of the side chains of the hydrophobic polymer chain segment of the block copolymer unit α have a hydrophilic group.

3. The polymer micelle pharmaceutical composition according to claim 1 or 2, containing 15 wt% to 80 wt% of the block copolymer unit α.

4. The polymer micelle pharmaceutical composition according to any one of claims 1 to 3, wherein the hydrophilic polymer chain segment of each of the block copolymer units α and β comprises a polyethylene glycol chain, and the hydrophobic polymer chain segment of each of the block copolymer units α and β comprises a polyamino acid chain.

5. The polymer micelle pharmaceutical composition according to any one of claims 1 to 4, further comprising a block copolymer unit γ having a hydrophilic polymer chain segment, which has a target binding site bound thereto, and a hydrophobic polymer chain segment,
   wherein the block copolymer unit γ is radially arranged together with the block copolymer units α and β in the state in which the hydrophilic polymer chain segment is directed outward and the hydrophobic polymer chain segment is directed inward.

6. The polymer micelle pharmaceutical composition according to any one of claims 1 to 5, wherein the block copolymer unit α has a lower hydrophobicity than the block copolymer unit β owing to the hydrophilic group (s) of the side chains of the hydrophobic polymer chain segment.

7. The polymer micelle pharmaceutical composition according to any one of claims 1 to 6, wherein:

   the hydrophobic polymer chain segment of the block copolymer unit β is constituted of repeating units having side chains; and
   the number of hydrophilic groups of the side chains of the hydrophobic polymer chain segment of the block copolymer unit α is larger than the number of hydrophilic groups of the side chains of the hydrophobic polymer chain segment of the block copolymer unit β.

8. The polymer micelle pharmaceutical composition according to any one of claims 1 to 7, wherein the block copolymer unit β has a drug bound thereto.

9. The polymer micelle pharmaceutical composition according to any one of claims 1 to 8, wherein the block copolymer unit α is free of a target binding site and a drug.

10. The polymer micelle pharmaceutical composition according to claim 8, wherein the block copolymer unit β has a

higher hydrophobicity than the block copolymer unit α owing to the drug.

**11.** The polymer micelle pharmaceutical composition according to claim 8, wherein the block copolymer unit β, even while having more hydrophilic groups than the block copolymer unit α, has a higher hydrophobicity than the block copolymer unit α owing to the drug.

FIG. 1

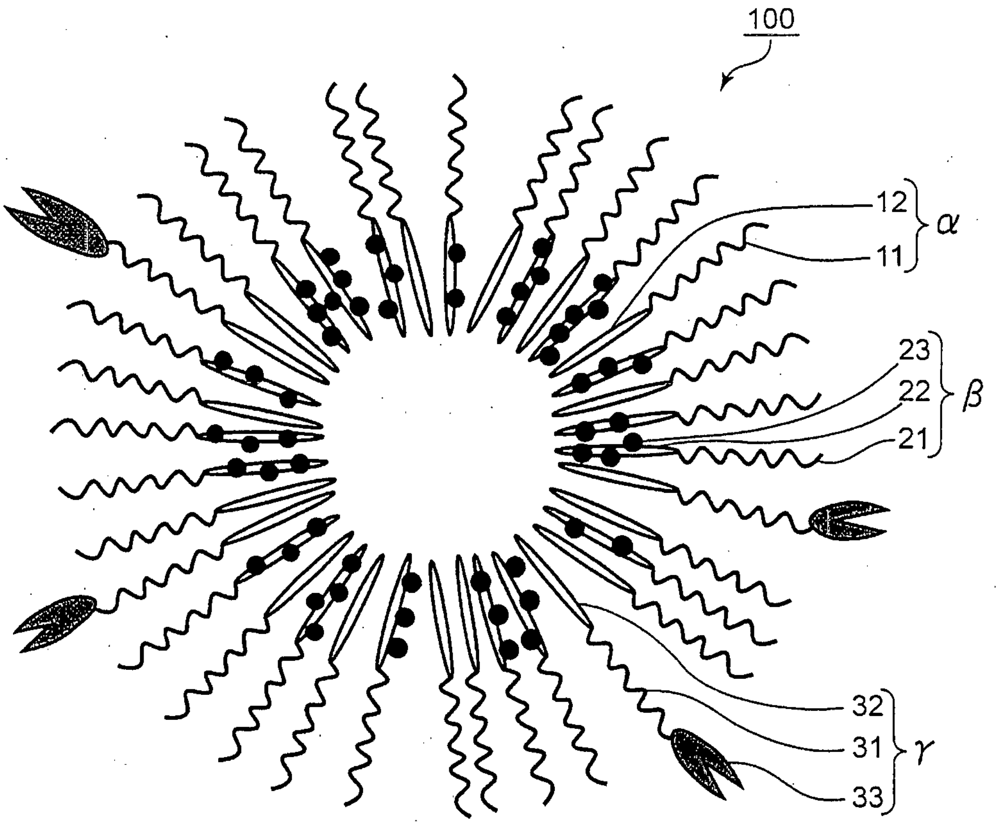

FIG.2

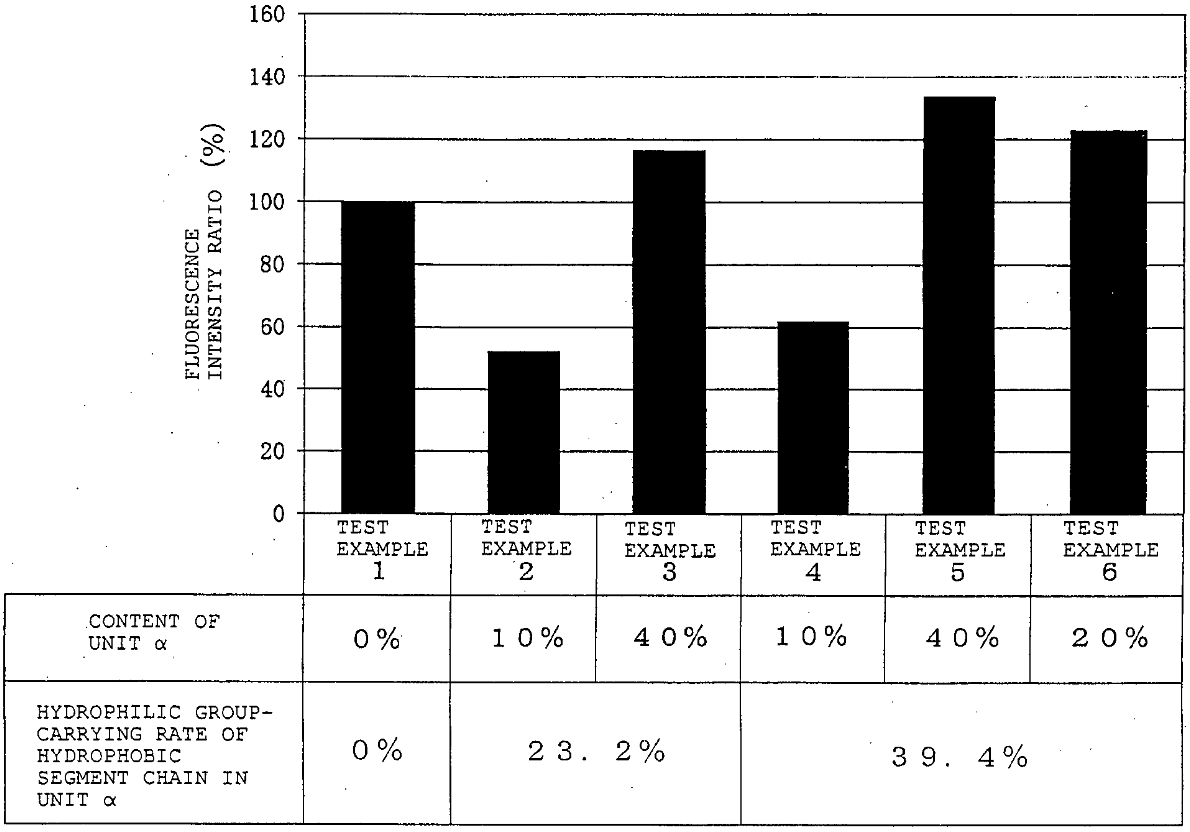


| | TEST EXAMPLE 1 | TEST EXAMPLE 2 | TEST EXAMPLE 3 | TEST EXAMPLE 4 | TEST EXAMPLE 5 | TEST EXAMPLE 6 |
|---|---|---|---|---|---|---|
| CONTENT OF UNIT α | 0% | 10% | 40% | 10% | 40% | 20% |
| HYDROPHILIC GROUP-CARRYING RATE OF HYDROPHOBIC SEGMENT CHAIN IN UNIT α | 0% | 23.2% | | 39.4% | | |

## INTERNATIONAL SEARCH REPORT

International application No.
PCT/JP2014/063020

A. CLASSIFICATION OF SUBJECT MATTER
*A61K47/34*(2006.01)i, *A61K9/107*(2006.01)i

According to International Patent Classification (IPC) or to both national classification and IPC

B. FIELDS SEARCHED

Minimum documentation searched (classification system followed by classification symbols)
A61K47/34, A61K9/107

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2011-162452 A (Nano Carrier Co., Ltd.), 25 August 2011 (25.08.2011), claims 1 to 2, 5 to 6 & US 2012/0093881 A1 & EP 2433618 A1 & WO 2011/096558 A1 | 1-11 |
| X | WO 2011/096558 A1 (Nano Carrier Co., Ltd.), 11 August 2011 (11.08.2011), claim 1 & US 2012/0093881 A1 & EP 2433618 A1 & JP 2011-162452 A | 1-11 |
| X | WO 2010/013836 A1 (Nano Carrier Co., Ltd.), 04 February 2010 (04.02.2010), claims 1 to 3 & JP 4538666 B2 & US 2010/0221320 A1 & EP 2281576 A1 | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>05 June, 2014 (05.06.14) | Date of mailing of the international search report<br>17 June, 2014 (17.06.14) |
| Name and mailing address of the ISA/<br>Japanese Patent Office<br><br>Facsimile No. | Authorized officer<br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009142326 A1
- WO 2010013836 A1
- WO 9633233 A1
- WO 9632434 A1
- WO 9706202 A1